(19)

Europäisches
Patentamt
European
Patent Office
Office européen
des brevets

(11) **EP 3 539 473 A1**

(12) **EUROPEAN PATENT APPLICATION**

(43) Date of publication:
**18.09.2019 Bulletin 2019/38**

(51) Int Cl.:
**A61B 5/1455** *(2006.01)* **A61B 5/00** *(2006.01)*
**A61B 5/1495** *(2006.01)*

(21) Application number: **19163299.1**

(22) Date of filing: **15.03.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2018 PL 42489718**

(71) Applicant: **TEX life&healthcare Sp. z o.o.
93-574 Lodz (PL)**

(72) Inventors:
• **Owczarek, Magdalena**
  **95-050 Konstantynów  ódzki (PL)**
• **Pabja czyk-Wlaz o, Ewelina**
  **93-351  ód (PL)**

(74) Representative: **Wroblewski, Michal
Kancelaria Patentowa
W.Sojka M.Wroblewski s.c.
ul. Gdanska 126/210
90-520 Lodz (PL)**

(54) **METHOD OF NON-INVASIVE MONITORING OF HYDRATION OF THE HUMAN BODY**

(57)    The solution according to the invention describes a method of measuring hydration of an organism, which consists in the evaluation system (2) activating optical sensors (3) which send signals to the wires (1). whereupon reflected light is captured by a photodiode (4). directed to evaluation system (2). and then through the interface (5). to the microcontroller (6). which activates the signaling diodes (8) or (10) and / or the display.

Fig. 2

EP 3 539 473 A1

**Description**

The subject of the invention is a method for non-invasive monitoring of the hydration of a human body.

The human body consists of an average of 60% water. 1% of weight loss due to fluid loss is defined as dehydration. The state of dehydration is very dangerous, especially for the elderly, children and athletes. It leads to the loss of strength, efficiency and consciousness, weakens immunity and also leads to irreversible damage to internal organs and even death. It is a state in which the body's water content falls below the value necessary for its proper functioning.

The body's water content depends mainly on sex, age and amount of adipose tissue. In new-borns it is about 75-80% of body weight, in a one-year-old child as much as 65%, while the total body water content of an adult human is 45-65% of body weight. The water content decreases with age, at the age of 60 it makes up approx. 50% of body weight.

Under normal physiological conditions person loses water during breathing, perspiration and urine excretion. If the loss of water from the body is not replenished, e.g. by drinking, then dehydration takes place. The initial phase of dehydration of the body is asymptomatic. As the degree of dehydration increases, numerous symptoms appear.

Normal water balance in the body is regulated by the sensation of thirst. In people over 50, thirst feeling decreases and decreases with age. Many elderly people suffer from symptoms resulting from dehydration. During the summer heat, progressive dehydration and hyperthermia often cause the death of older people.

In the case of a healthy human body, water shortage is manifested by a thirst, but in some cases this symptom is distorted or difficult to observe (especially in children and the elderly). Typically, clinical assessment of the level of hydration is based on physical examination. Symptoms of dehydration include dry mouth and mucous membranes, sunken eyes, orthostatic hypotension, a weak skin turgor. These symptoms are often diagnosed in a medical examination. However, clinical assessments may be subjective, can be of low sensitivity and specificity. Laboratory tests of blood samples, creatinine in serum and urine are also used to determine the state of dehydration. The main advantage of these tests compared to physical examination is that they provide objective and quantitative measurements. Nevertheless, they require special laboratory equipment to be invasive and usually time-consuming. There is therefore a need for a rapid and non-invasive method that would overcome the above-mentioned drawbacks.

The method for assessing the hydration level is described in patent application EP2004051A1. The measurement consists of the use of the mechanical wave transmitted by the transmitter to the tissue being examined, which, after reflection, returns to the transducer. The assessment of the hydration level takes place by assessing the measurement of the difference in the speed of the mechanical wave transmitted in the direction of the examined tissue and the reflected wave.

Patent GB201209340 describes a method for measuring hydration by measuring the content of a selected chemical substance, such as calcium, zinc, bicarbonate, sodium chloride, lactic acid, urea in sweat. The measurement is carried out colorimetrically.

The patent US7033321B1 describes the measurement of hydration by determining the velocity of the ultrasound pulses through the examined tissue.

Patent application US20130144136A1 describes the monitoring of the hydration level of the patient using spectroscopic technique. The measuring system consists of a microprocessor and a sensor system with a light source and a light detector. The microprocessor connected functionally to the light source and the light detector controls the operation of the light source. The light is emitted by the light source towards the tissue of the patient being examined. It passes through or is reflected by the tissue and is then picked up by the light detector. The light detector transmits the measurement of light intensity received by the detector to the microprocessor. The microprocessor is programmed using a tissue hydration model that uses the light intensity measurement to determine the hydration value of the patient's tissue. The light source can be any suitable light source, such as an incandescent source or a light-emitting diode.

The method of non-invasive monitoring of the hydration of a human body according to the invention is based on the use of venous blood property consisting in a change in its colour depending on the density and the degree of hydration of the body.

Blood colour is given by erythrocytes containing the red dye, the so-called haemoglobin. However, the red colour of blood may have different shades. The degree of colour varies depending on the degree of oxygenation of the blood and the degree of hydration of the body. Oxygen-carrying blood is defined as arterial and has a bright red colour. When oxygen gets rid of it, it turns dark red and is called a venous blood.

The number of erythrocytes in the blood is indicated by the index referred to as haematocrit. It allows you to determine what percentage of your entire blood volume are erythrocytes. The classic method of determining the haematocrit index is a microscopic or micro-nucleotide method, based on controlled blood centrifugation in a calibrated capillary. To test the haematocrit level, a blood sample is collected from the ulnar vein or fingertip.

The level of haematocrit increases when the erythrocytes are overproduced in the blood and are densified. Elevated levels of haematocrit most often occur in the dehydration of the body, with primary anemia, polyglobule, and polycythemia vera.

A decrease in the haematocrit level may be evidence of hyperhydration or anemia (anemia). It also follows bleeding (morphotic components are slower to reproduce)[4].

The correct haematocrit indicators are as follows:

- women: 37-47%

- men: 42-52%

- newborns: 44-62%

- for infants and children: 30-40%

A method for measuring haemoglobin and/or haematocrit levels in blood is described in patent application WO2002015787A1. Measurements are carried out using a spectral method using scattered light. In this solution, a linear relationship between the haematocrit content and the density of blood was used.

The method for monitoring hydration of an organism according to the invention consists in the optical measurement of a signal which is information about the colour of venous blood resulting from the level of haematocrit.

In each case of an increase in the hydration decline, the following relationship occurs: the density of the blood increases, the heart rate increases, the pressure drops, the blood becomes darker.

In order to conduct the measurement, the sensor is attached to the tested site, which is a specific part of the body according to the selected type of measurement. It can be integrated, for example, with a band, which is placed on different parts of the body (in particular on the wrist, calf, head, chest), with a patch on the skin, a clip (for example on the ear) or in the form integrated with clothing or other textiles.
The evaluation system which is one of the elements of the optical system launches optical sensors, preferably diodes highlighting LED (infrared - IR, red - RED or green - GREEN), which output signals reaching through the skin into veins. The reflected signals are captured by an optical sensor, preferably a diode, most preferably a photodiode, and directed to the evaluation system, which determines the level of the signal and calculates the level of hydration. The signal from the evaluation system is directed through the interface to the microcontroller which, depending on the obtained result, launches green or red signalling diodes and/or a display giving information in the form of a digital value informing the tester about the state of hydration body, while in the first stage of the method according to the invention, before the actual measurement, it personalizes an algorithm for a given person with the use of the computing device, i.e. for a period of several days a calibration is carried out, the pulse and temperature are read from diode indications and taking into account individual features such as age, sex, activity , body mass, blood group, frequency of drinking. On this basis, the algorithm carries out measurements, which are than approximated to the levels of extreme changes for a given individual.

In the second stage it enters the continuous mode with adjustable frequency of measurement, favourable for people being more physically active, less for people with sedentary mode. The measurement is generated at a specific frequency, the device calculates average values and signals the information about the hydration level.

The above-described method of the invention enables a non-invasive, optical measurement of hydration of the body.

Examples of implementation

The solution according to the invention is shown in the embodiments and in the figure, fig. 1 shows the wristband with visible optical system, fig. 2 - the optical path of the signal waveform, fig. 3 - a block diagram of the electronic circuitry of the band, fig. 4-9 conditions and measurement results, fig. 10-12, conditions and results of comparative measurements on venous blood samples.

In the conducted tests, an evaluation system was used to measure the level of blood oxygenation, which is used for measuring and signal conditioning, controlling of the light-emitting diodes and sending the converted signal to the microcontroller. The system communicates via the SPI interface. The system also includes a 32 bit microcontroller and a power source.

In order to conduct the measurement of the tested place, a sensor with an optical system is attached. The evaluation system 2 activates optical sensors, preferably LED 3 irradiating diodes, which send, reaching through the skin to the veins 1, signals of a wavelength of 450-600 nm and/or 630-690 nm and/or 780-1100 nm. The 4 reflected signals captured by the photodiode are sent to the evaluation system 2, and then via the SPI 5 interface, to the microcontroller 6 equipped with green 8 and red 10 on the display informing the individual about the hydration level and the power source not shown in the diagram.

In the first step of the method according to the invention, the algorithm is personalized for a particular individual through initial pre-calibrating of the device for several days, taking into account individual features such as age, sex, activity, body weight, blood group, drinking frequency, while measuring its temperature and pulse. After the calibration is completed, the correct measurements are launched in the continuous mode with the possibility of setting the measurement frequency, greater for people who are active in sports, less for people with sedentary mode. The measurement is generated at a specific frequency, the device calculates average values and displays information about the hydration level.

Example 1

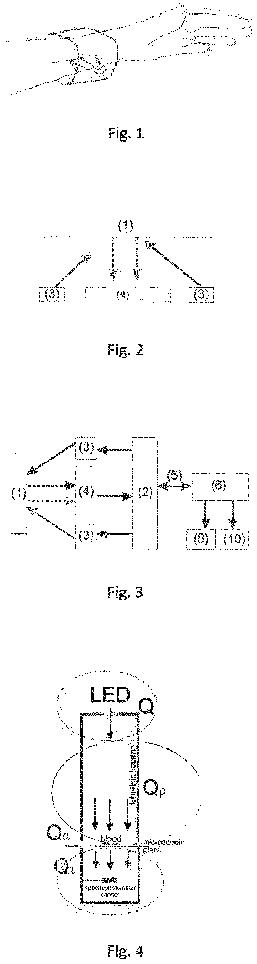

The measurement was conducted on the electronic evaluation system using IR and RED diodes on the finger on 4 people in the experiment from the state of dehydration through the state of gradual hydration; The measurement conditions and results are presented in Table 1 and in Figure 1. The variability of the IR signal was observed depending on the change in hydration for all measured persons, whereas for the RED signal in half the cases. The increase in the signal level was dependent in time on the gradual intake of liquids and a decrease on their excretion.

Table 1

| Case | | avarage values | | |
|---|---|---|---|---|
| sample name | time | RED [V] | IR [V] | Fluid intake [l] |
| | 09:50 | 0.108 | 0.102 | 0 |
| | 10:39 | 0.107 | 0.132 | 1 |
| 1_NAW_1 | 11:41 | 0.106 | 0.130 | 0 |
| | 09:48 | 0.080 | 0.072 | 0 |
| | 10:45 | 0.089 | 0.088 | 0.5 |
| 1_NAW_2 | 11:38 | 0.085 | 0.086 | 0.5 |
| | 09:46 | 0.084 | 0.074 | 0 |
| | 10:36 | 0.084 | 0.083 | 0.5 |
| 1_NAW_3 | 11:39 | 0.082 | 0.078 | 0 |
| | 09:44 | 0.066 | 0.052 | 0 |
| | 10:41 | 0.079 | 0.069 | 0.5 |
| 1_NAW_4 | 11:44 | 0.070 | 0.056 | 0 |

where:

1_NAW_1, 1_NAW_2, 1_NAW_3, 1_NAW_4 -the first round of tests of body hydration (from the state of dehydration to the state of hydration) on a sample composed of 4 people

RED, IR [V] - transmission diodes, optical measurement on the finger

Figure 1

where:
1_NAW_1, 1_NAW_2, 1_NAW_3, 1_NAW_4 - the first round of tests of body hydration (from the state of dehydration to the state of hydration) on a sample composed of 4 people
RED, IR [V] - transmission diodes, optical measurement on the finger

Example 2

The research was carried out by the use of electronic evaluation system using IR and RED diodes in the case of a finger test and GREEN diodes in the case of wrist testing. In the case of testing on the wrist, a measuring band is placed on the wrist in such a way that the optical system is located on the inside of the hand with visible veins. The experiment was carried out on persons: from the state of dehydration through the state of gradual hydration, and from the state of hydration to the state of dehydration (exercise test on a cycling exercise) in laboratory conditions, in the confrontation of optical measurement with laboratory parameters. The measurement conditions and results are shown in Table 2 and Chart 2 for the case of dehydration by exercise and in Table 3 and in Chart 3 for the case of gradual hydration from the dehydration status.

The variability of the RED, IR and GREEN signal was observed depending on the change of the degree of hydration to the state of dehydration (during exercise). For the GREEN signal, this variation is consistent with the level of intake and excretion of water in time. For the IR, RED signal, a diametric decline in hydration during exercise and a minimal increase during water intake were observed. In contrast, laboratory parameters (body weight and hydration by impedance) indicate the variability of hydration in time, however not instantaneously as the GREEN optical measurement.

Podobnie zaobserwowano zmiennosc sygnatu RED, IR i GREEN w zaleznosci od zmiany stopnia odwodnienia do stanu nawodnienia. Dla sygnatu GREEN zmiennosc ta jest zgodna i natychmiastowa z poziomem przyjmowanej wody w czasie. Dla sygnatu IR, RED obserwowano podobną zmiennosc. Natomiast parametry laboratoryjne: masa ciata obrazuje zmiennosc nawodnienia, a parametr nawodnienia metodą impedancji zupetni nie obrazuje zmiennosci zaleznej od procesu nawadniania organizmów.

Similarly, the variability of the RED, IR and GREEN signal was observed depending on the change of the degree of dehydration to the state of hydration. For the GREEN signal, the variability is consistent and immediate with the level of water taken in time. A similar variability was observed for the IR, RED signal. On the other hand, laboratory parameters: body mass illustrates the variability of hydration, while the hydration measurement by the impedance method does not show the variability depending on the hydration process.

Table 2

| 2_ODW_1- from the state of hydration to the state of dehydration (effort 30 minutes to 15:17) | | | | | | | |
|---|---|---|---|---|---|---|---|
| time | 14:35 | 15:17 | 15:40 | 16:00 | 16:20 | 16:40 | 17:00 |
| water [l] | 2 (morning) | effort | 0.2 | 0.2 | 0.2 | 0.2 | WC |
| m.c. [kg] | 88.6 | 88.2 | 88.1 | 88 | 88 | 88 | 87.3 |
| Fat [%] | 26.4 | 24.7 | 25.1 | 25.4 | 25.8 | 25.7 | 25.2 |
| Naw [%] | 52.1 | 53.6 | 53.2 | 52.9 | 52.6 | 52.7 | 53.1 |
| m.m. [kg] | 62.0 | 63.1 | 62.7 | 62.4 | 62.1 | 62.1 | 62.1 |
| IR [V] | 0.03 | 0.079 | 0.062 | 0.024 | 0.024 | 0.032 | 0.024 |
| RED [V] | 0.026 | 0.101 | 0.084 | 0.026 | 0.026 | 0.031 | 0.028 |
| Green [V] | 0.033 | 0.397 | 0.796 | 0.853 | 0.822 | 0.86 | 0.755 |

where:

2_ODW_1- the second round of tests of body hydration (from the state of hydration to the state of dehydration) during physical effort 30 min.
RED, IR - transmission diodes, optical measurement on the finger
GREEN - transmission diode, optical measurement on the wrist
Time [h] - time of measurement
Fluids [l] - fluid intake
b.w.. [kg] - body weight, measurement in the laboratory
Fat [%] - fat content, measurement in the laboratory
Hyd[%] - impedance method, measurement in the laboratory
m.m.[kg] - muscle mass, measurement in the laboratory

## Figure 2

where:
2_ODW1_IR, RED - the result of the hydration measurement by means of IR and RED diodes (from the hydrated state to the state of dehydration) during an effort of 30 min, optical measurement on the finger,
2_ODW1_GREEN - the result of hydration measurement using the GREEN diode (from the hydrated state to the state of dehydration) during an effort of 30 min, optical measurement on the wrist,
2_ODW1_Hyd% LAB - the result of hydration measurement by impedance method (from the hydrated state to the state of dehydration) during 30 min exercise, measurement on a laboratory scale,
2_ODW1_m.c. [kg] LAB - the result of hydration measurement by body mass measurement (from the hydrated state to the state of dehydration) during 30 min exercise, measurement on a laboratory scale

**Table 3**

| 2_NAW1 from the dehydrated state to the state of hydration | | | | | |
|---|---|---|---|---|---|
| time | 14:50 | 15:28 | 16:00 | 16:22 | 16:50 |
| water [l] | 0 | 0.5 | 0.5 | 0 | WC |
| m.c. [kg] | 65.3 | 65.8 | 66.2 | 66.2 | 65.9 |
| Fat [%] | 29.4 | 29.8 | 29.7 | 30.4 | 29.7 |
| Naw [%] | 51.9 | 51.7 | 51.7 | 51.2 | 51.7 |
| m.m. [kg] | 43.8 | 43.9 | 44.2 | 43.8 | 44 |
| **IR [V]** | **0.065** | **0.098** | **0.104** | **0.096** | **0.095** |
| **RED [V]** | **0.555** | **0.107** | **0.111** | **0.088** | **0.117** |
| **Green [V]** | **0.583** | **-** | **1.022** | **0.954** | **1.073** |
| 2_NAW2 from the dehydrated state to the state of hydration | | | | | |
| time | 14:56 | 15:30 | 15:56 | 16:24 | 16:55 |
| water [l] | 0 | 0.5 | 0.5 | 0.5 | WC |
| m.c. [kg] | 74.8 | 75.2 | 75.7 | 75.7 | 75.6 |
| Fat [%] | 34.4 | 34.8 | 35.2 | 34.9 | 34.6 |
| Naw [%] | 48.9 | 48.6 | 48.4 | 48.6 | 48.8 |
| m.m. [kg] | 46.6 | 46.5 | 46.6 | 46.8 | 46.9 |
| **IR [V]** | **0.066** | **0.074** | **0.075** | **-** | **0.059** |
| **RED [V]** | **0.711** | **1.023** | **0.917** | **-** | **0.069** |
| **Green [V]** | **0.119** | **1.023** | **0.968** | **-** | **1.065** |

Where:
2_NAW_1, 2_NAW_2- the second round of tests of body hydration (from the state of dehydration to the state of hydration)
RED, IR-transmission diodes, optical measurement on the finger GREEN - transmission diode, optical measurement on the wrist
Time [h] - time of measurement
Fluids [l] - fluid intake
b.w.. [kg] - body weight, measurement in the laboratory
Fat [%] - fat content, measurement in the laboratory
Hyd[%] - impedance method, measurement in the laboratory
m.m.[kg] - muscle mass, measurement in the laboratory

Figure 3

where:

2_NAW1_IR, RED, 2_NAW2_IR, RED - the result of hydration measurement of organisms using IR and RED diodes (from the state of dehydration to the state of hydration), optical measurement on the finger,

2_NAW1_GREEN, 2_NAW2_GREEN - the result of hydration measurement of organisms using the GREEN diode (from the state of dehydration to the state of hydration), optical measurement on the wrist,

2_NAW1_Hyd% LAB, 2_NAW2_NAW% LAB - the result of hydration measurement of organisms by impedance (from the state of dehydration to the state of hydration), measurement on a laboratory scale,

2_NAW1_b.m. [Kg] LAB - the result of hydration measurement of organisms using the method of measuring body weight (from the state of dehydration to the state of hydration), measurement on a laboratory scale, 2_NAW1_woda, 2_NAW2_woda - the hydration process, water intake

**Example 3**

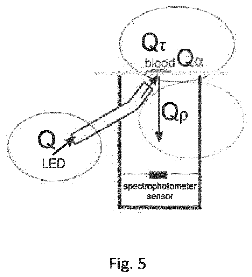

Comparable testing was conducted on blood samples. The tests were carried out directly on venous blood samples to determine the optical properties of blood with different degrees of hydration using a spectrophotometer in laboratory conditions. The tests were carried out in two variants: transmitted light (Figure 4) and venous blood reflected from the sample (Figure 5), on which

$$Q\rho = Q-(Q\tau+Q\alpha)$$

$$Q\rho+Q\tau+Q\alpha=1$$

**Q$\rho$- component of the reflection**
Q$\alpha$ - component of absorption
Q$\tau$ - component of transmission

The results are presented in Figure 4. The parameters obtained by the spectrophotometer Ra, R9, CCT exhibit variability - sensitivity to changes in the blood concentration in the sample, along with the increase in the saline concentration in the blood in both variants of light. This variability follows a characteristic upward or downward trend, except for the measurement of 88% blood concentration (12% saline) or for 82% blood concentration (18% saline) which disrupts this trend in a given test variant, which may indicate nonlinearity of this phenomenon or the effect of blood absorption and fluorescence properties. The R9 parameter - partial red colour rendering index shows that with increasing saline concentration in the blood the value of this coefficient in the reflection component decreases (variant of reflected light). However, in the transmission component (variant of transmitted light), the R9 value increases with increasing saline concentration in the blood, with the exception of the light bulb.

In the case of the proposed method, the reflection component is very important for the measurement of hydration. The value of parameter R9 indicates the degree of red colour rendering, which may be the degree of hydration of the body.

## Figure 4

transmitted light reflected light

where:

R9_Prz W2, R9_Prz Light bulb, R9_Frz NGreen - measurement result of R9 fractional red colour coefficient using LED W2 (408-850 [nm]), Light bulbs (380-850 [nm]), NGreen diodes (450-600) in transmitted light

R9_Odb W2, R9_Odb Light Bulb, R9_Odb NGreen, R9_Odb RED - the R9 measurement result of the partial red colour coefficient using the W2 LED (408-850 [nm]), Light Bulbs (380-850 [nm]), NGreen (450-600), Red diodes (630-690 [nm]) in reflected light

Table 4

| Blood concentration [%] | 0 | 100 | 94 | 88 | 82 |
|---|---|---|---|---|---|
| Transmitted light | | | | | |
| **R9_Prz W2 [ ]** | **-7.6** | **0.7** | **5.0** | **-3.3** | **14.6** |
| **R9_Prz Żarówka [ ]** | **83.6** | **69.5** | **51.3** | **58.6** | **47.0** |
| **R9_Prz NGreen [ ]** | **25.8** | **21.1** | **20.3** | **20.7** | **63.5** |
| Ra_Prz W2 [ ] | 75.4 | 76.6 | 77.0 | 75.9 | 78.6 |
| Ra_Prz Zar6wka [ ] | 96.5 | 93 | 88.3 | 89.8 | 87.3 |

(continued)

| Transmitted light | | | | | |
|---|---|---|---|---|---|
| Ra_Prz NGreen [ ] | 79.5 | 78.4 | 78.6 | 78.5 | 86.7 |
| CCT_Prz W2 [K] | 5152 | 5026 | 4927 | 5020 | 4709 |
| CCT_Prz Żarówka [K] | 2837 | 2757 | 2651 | 2669 | 2779 |
| CCT_Prz NGreen [K] | 8769 | 8659 | 8651 | 8622 | 9027 |
| **Reflected light** | | | | | |
| **R9_Odb W2 [ ]** | **-27.8** | **97.3** | **62.2** | **36.9** | **75.2** |
| **R9_ Odb Żarówka [ ]** | **94.1** | **24.30** | **20.90** | **-44.6** | **29.10** |
| **R9_ Odb NGreen [ ]** | **81.9** | **63.1** | **52.1** | **58.9** | **-45.2** |
| Ra_Odb W2 [ ] | **72.1** | **91.1** | **86.0** | **81.7** | **92.1** |
| Ra_Odb Żarówka [ ] | **98.6** | **80.4** | **73.5** | **64.1** | **79.9** |
| Ra_ Odb NGreen [ ] | **81.1** | **74.6** | **72.2** | **73.5** | **-42.1** |
| CCT_Odb W2 [K] | **4941** | **3574** | **4054** | **4731** | **3353** |
| CCT_Odb Żarówka [K] | **2918** | **2181** | **1888** | **2245** | **2201** |
| CCT_Odb NGreen [K] | - | - | - | - | - |

where:
Selected parameters from the spectrophotometer:
R9 [] partial colour rendering index
Ra [] CRI colour rendering index
CCT [K] colour temperature
For light sources:
W2 - White LEDs (408-850 [nm]).
Light bulbs (380-850 [nm]).
NGreen diodes (450-600)
Red diodes (630-690 [nm])

Example 4

Description

The research was carried out on electronic evaluation systems using RED. Green and IR diodes in the following diode sets of the given system: G. GG. RG. GR. RIR. The study was carried out on the wrist. A wristband is worn on the tested wrist in such a way that the optical system is located on the inside of the hand with visible veins. The study was conducted on 40 athletes of amateur athletes - male riders.

Test procedure

The experiment was conducted from the state of hydration to the state of dehydration in laboratory conditions (air-conditioned sports and medical laboratory). in the confrontation of optical measurement with invasive medical tests - i.e. analytical tests of blood collected from participants and laboratory parameters. Invasive tests were carried out - blood tests before and after exercise testing and determination of physiological parameters (height. weight. age. body composition). The measurement conditions and results are shown in Table 5 and Figure 5.

The test procedure consisted of carrying out a stress test on the exercise bike during 60 minutes. The load of participants during the test was determined on the basis of the individual performance threshold. Each participant during the study was monitored on-line in terms of performance and vital parameters.

Observations

The variability of the RED. IR and GREEN signal was observed depending on the change in the degree of hydration during the stress test (Figures 5-8). For GREEN signal. this variability is observed immediately and is equal to the amount of water excreted [in%] as a result of sweating of the body. which occurs during an exercise test lasting 60 minutes (Figure).

For the RED signal. the trend line of RED signal variation was most often observed during the process of effort. Similar variability was observed for the IR signal.

The combination of diodes: R. IR. GREEN into double systems: GG. RG. GR. RIR does not change the characteristics of individual diode signals.

On the other hand. laboratory parameters determining the physiological parameters of a person: body weight. show the variability of hydration. and the body composition parameters. measured by impedance (water. muscle. fat) after an exercise test. do not indicate an adequate hydration change. Measurement of hydration by impedance after exercise test shows an increase in hydration. which is not consistent with the decrease in hydration noted by the blood morphology parameters and the tested sensors. Hydration indices marked from invasive blood counts. performed before and after the study. confirmed the variability (decrease) of hydration during the exercise test. Elevated haemoglobin (Hb) and haematocrit (HCT). urea / nitrogen (BUN) and creatinine were observed.

Osmolality of the plasma also increases. which in the following tests was calculated from the following formula:

$$[mOsm / kg \, H2O] = 2 \times [Na +] \, [mmol / l] + glucose \, [mmol / l] + urea \, [mmol / l]$$

Invasive tests in the form of blood tests and changes in body weight before and after stress tests confirmed compliance with signal variability on sensors on electronic evaluation systems using diodes. especially Green diodes (RED. Green and IR). With the decrease of hydration during the stress test. the Green diode chart adopts a downward trend. The level of the trend factor for this G diode is -2.1668E-06. Similarly. the IR diode. although to a lesser extent than the Green diode. the value of the trend line coefficient is-1.1E-06. However. R diodes in each system have an upward trend. the trend factor is 3.314E-06.

The process that takes place in the body during the stress test also shows the temperature measurement. which indicates an upward trend on average from 29.75°C to 34.00 °C.

Table 5

| Physiological parameters | | Average | Standard deviation | min. | max |
|---|---|---|---|---|---|
| Height. b.m. | Age | 31.71 | 8.01 | 17.00 | 48.00 |
| | Height [cm] | 179.03 | 5.93 | 162.00 | 194.00 |
| | BMI | 23.92 | 1.74 | 21.20 | 29.30 |
| | b.m. before [kg] | 76.71 | 7.23 | 60.40 | 92.90 |
| | b.m. after [kg] | 75.29 | 7.13 | 59.10 | 91.50 |
| | b.m. loss. [%] | 1.86% | 0.35% | 1.04% | 2.89% |
| Impedance method | FAT before [%] | 13.48% | 3.55% | 6.20% | 22.30% |
| | FAT after [%] | 9.04% | 2.96% | 4.10% | 16.70% |
| | m.mś. przed[kg] | 62.96 | 5.06 | 50.40 | 76.10 |
| | m.mś. po [kg] | 64.78% | 5.49% | 52.10% | 79.40% |
| | Hydration before [%] | 62.08% | 3.09% | 54.70% | 70.60% |
| | Hybdration after [%] | 66.20% | 3.01% | 61.00% | 75.70% |
| | **Hydration loss. [%]** | **-6.74%** | **2.04%** | **-11.52%** | **-3.34%** |
| blood morphology before | Hb | 15.73 | 0.78 | 14.40 | 17.30 |
| | HCT | 41.57 | 12.21 | 0.42 | 49.10 |
| | Osmolality | 293.62 | 3.99 | 287.17 | 303.51 |
| | urea | 34.35 | 8.60 | 20.90 | 55.70 |
| | BUN | 16.05 | 4.02 | 9.80 | 26.00 |
| | creatytnine | 0.93 | 0.14 | 0.76 | 1.49 |
| | Glu | 92.03 | 22.42 | 58.00 | 161.00 |
| | Na | 140.78 | 1.97 | 137.00 | 145.00 |
| blood | Hb | 16.38 | 0.79 | 14.90 | 18.00 |

|  |  |  |  |  |  |
|---|---|---|---|---|---|
|  | HCT | 0.47 | 0.02 | 0.43 | 0.52 |
|  | Osmolality | 298.54 | 3.17 | 291.44 | 304.34 |
|  | urea | 38.20 | 9.13 | 24.50 | 62.60 |
|  | BUN | 17.59 | 4.77 | 4.00 | 29.30 |
|  | creatynine | 1.31 | 0.16 | 1.04 | 1.77 |
|  | Glu | 103.34 | 21.65 | 78.00 | 190.00 |
|  | Na | 142.58 | 1.63 | 139.00 | 145.00 |
| Optical measurment | Temp. [degC] przed | 29.74 | 2.54 | 21.47 | 32.70 |
|  | Temp. [degC] po | 34.00 | 0.94 | 31.75 | 35.24 |
|  | R [V] przed | 0.0079 | 0.0064 | 0.0018 | 0.0206 |
|  | R [V] po | 0.0149 | 0.0049 | 0.0047 | 0.0222 |
|  | Trend coeff. R | 3.314E-06 | 2.01021E-06 | 3.722E-07 | 8.0521E-06 |
|  | G [V] before | 0.0193 | 0.0108 | 0.0006 | 0.0349 |
|  | G [V] after | 0.0135 | 0.0070 | 0.0004 | 0.0226 |
|  | Trend coeff. G | -2.168E-06 | 3.10115E-06 | -1.24127E-05 | 3.3067E-06 |
|  | IR [V] before | 0.0230 | 0.0049 | 0.0136 | 0.0280 |
|  | IR [V] after | 0.0179 | 0.0044 | 0.0127 | 0.0256 |
|  | Trend coeff. IR | -1.1E-06 | 7.88295E-07 | -2.1625E-06 | -6.85E-08 |

Where:

b.w. [kg] - body weight. laboratory measurement (impedance method), before and after the test.

FAT [%] - fat content. laboratory measurement (impedance method). before and after the test

m.m. [kg] - muscle mass. laboratory measurement (impedance method). before and after the test

Hyd. before [%] - hydration. measurement in the laboratory (impedance method). before and after the test

Hb Hemoglobin - (blood morphology, before and after the test). red blood pigment, protein contained in erythrocytes. whose essential function is transporting oxygen - attaching it to the lungs and release in the tissues reference values: 11-17.5 g/dl

HCT Haematocrit - (blood morphology, before and after the study), the ratio of erythrocyte volume to whole blood volume. elevated level most often testifies to isotonic dehydration reference values: 42-52%.

Serum osmolality - (blood morphology, before and after the test) the number of osmols (moles of osmotically active substances) dissolved in one kilogram of solvent (water) reference values: <60 years 275-295 mOsmol / kg H2O.

Urea - (blood morphology, before and after the test) is mainly produced in the liver. The concentration of urea in the serum allows to determine whether the kidneys perform their excretory function (suspected renal failure). For the study to be unambiguous and reliable. it should be combined with the measurement of creatinine (the ratio of urea to creatinine in the blood serum). Thanks to the examination, it is also possible to determine whether a given patient has catabolic states and dehydration.

BUN - (blood morphology, before and after the test) urea / nitrogen ratio of urea.

Creatinine - (blood morphology. before and after the test) organic chemical compound excreted by the kidneys. It occurs in blood serum and in urine. It is a nitrogen compound and is a product of changes in skeletal muscle. so it is a product of metabolism. Creatinine is a marker that allows us to monitor the functioning of the kidneys, reference values: 0.6-1.3 mg / dl

Glu - glucose. (blood counts, before and after the test).

Na - sód (blood counts, before and after the study).

Temp. [°C] before the "on line" temperature measurement. the initial value from the exercise test.

Temp. [°C] after - temperature measurement "on line" the final value of the exercise test.

R [V] before. G [V] before. IR [V] before - transmission diodes, optical measurement on the wrist "on line". initial value from the stress test.

R [V] po. G [V] po. IR [V] post-transmission diodes. optical measurement of the wrist on-line. final value of the exercise test.

**trend coeff. R. trend coeff. G. trend coeff. IR** - coefficient a of the determined trend line from the optical measurement of a given transmission diode

Figure 5 The result of the measurement of hydration change in the body carried out using RED and Green LEDs. RG sensor.

where:
RG: R_. RG: _G [V] - the result of the measurement of the hydration change of the body using RED and Green LEDs (from the hydrated state to the state of dehydration) during an exercise test of 60 min. optical measurement on the inside wrist site. study start 11.06. anaerobic threshold at 11.21. stop test hours 12.06.

Temperature [°C] - temperature measurement result during an exercise test of 60 min.

Figure 6 The result of the measurement of the hydration change carried out with the use of two Green diodes. the GG sensor

where:

GG: G_. GG: _G [V] - the result of the measurement of the hydration change of the body using two Green diodes (from the hydrated state to the state of dehydration) during an exercise test of 60 min. optical measurement on the inside wrist side. start test hours 8.47. anaerobic threshold 9.02. stop test hours 9.47.

Temperature [°C] - temperature measurement result during an exercise test 60 min.

Figure 7 The result of the measurement of the hydration change of the organism carried out with RED and IR diodes. RIR sensor.

where:

RIR: R_. RIR: _IR [V] - the result of the measurement of the hydration change by means of RED and IR LEDs (from the hydrated state to the state of dehydration) during an exercise test of 60 min. optical measurement on the inside wrist site. study start 11.22. anaerobic threshold at 11.36. stop test hours 12.22.

Temperature [°C] - temperature measurement result during an exercise test of 60 min.

Figure 8 The result of the measurement of hydration change in the body carried out using RED and Green LEDs. GR sensor.

where:
GR: R_. GR: _G [V] - the result of the measurement of the hydration change of the body using RED and Green LEDs (from the hydrated state to the state of dehydration) during an exercise test of 60 min. optical measurement on the inside wrist site. study start 9:22. anaerobic threshold. 9:38. stop of the test hours 10.22.

Temperature [°C] - temperature measurement result during an exercise test of 60 min.

**Claims**

1. A method of non-invasive monitoring of body hydration by means of an optical sensor attached to the body. **characterized by** the fact that the optical sensors (3) triggered by the evaluation system send signals through the skin to the veins (1). after which the reflected light captured by the optical sensor (4). is directed to the evaluation system (2). and then via the interface (5). to the microcontroller (6). which. depending on the obtained result. activates the signalling diode (8) or (10) and / or provides information in the form on the display. while the device is calibrated before the first measurement and determines the algorithm for the person being tested.

2. The method according to claim 1. **characterized in that** the temperature and pulse are measured at the device calibration stage. taking into account individual data such as age. sex. activity. body weight. blood group. frequency of drinking.

3. Method according to claim 1 or 2. **characterized in that** the pulse measurement is calculated from the diode signals (8) and (10).

4. The method according to claim The method of claim 1 or 2. wherein the temperature is indicated by a thermometer mounted on the optical sensor.

5. The method according to claim 1. **characterized in that** the optical sensors (3) are LED diodes.

6. Method according to claim The system of claim 1 or 2. **characterized in that** the sensor comprises at least one LED.

7. Method according to all the above claims. **characterized in that** the diode (3) emits light with a wavelength of 450-600 nm and / or 630-690 nm and / or 700-1100 nm.

8. Method according to claim 1. **characterized in that** the optical sensor (4) is a diode.

9. Method according to claim The method of claim 1 or 5. **characterized in that** the optical sensor (4) is a photodiode.

Fig. 1

Fig. 2

Fig. 3

Fig. 4

Fig. 5

Europäisches
Patentamt

European
Patent Office

Office européen
des brevets

# EUROPEAN SEARCH REPORT

Application Number

EP 19 16 3299

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/088002 A1 (PODHAJSKY RONALD [US] ET AL) 26 March 2015 (2015-03-26)<br>* paragraph [0004] *<br>* paragraph [0015] *<br>* paragraph [0024] - paragraph [0026] *<br>* paragraph [0033] *<br>* paragraph [0036] *<br>* paragraph [0040] *<br>* paragraph [0043] *<br>* paragraph [0044] *<br>* paragraph [0045] *<br>* paragraph [0069] - paragraph [0070] *<br>* 304, 306, 308, 316, 310, 312, 324, 330; figure 3 *<br>----- | 1-9 | INV.<br>A61B5/1455<br>A61B5/00<br>A61B5/1495 |
| X | US 2013/144136 A1 (RYMUT RUSSELL [US]) 6 June 2013 (2013-06-06)<br>* paragraph [0060] - paragraph [0061] *<br>* paragraph [0025] *<br>* paragraph [0103] - paragraph [0104] *<br>* paragraph [0095] - paragraph [0096] *<br>* paragraph [0105] *<br>* paragraph [0068] *<br>----- | 1,2,5,6, 8,9 | |
| A | US 2015/289820 A1 (MILLER DEVIN WARNER [US] ET AL) 15 October 2015 (2015-10-15)<br>* paragraph [0092] *<br>----- | 2,7 | TECHNICAL FIELDS SEARCHED (IPC)<br><br>A61B |
| A | US 2017/360316 A1 (GU SHIQUN [US] ET AL) 21 December 2017 (2017-12-21)<br>* paragraph [0021] *<br>* paragraph [0024] *<br>----- | 7,9 | |

The present search report has been drawn up for all claims

| Place of search | Date of completion of the search | Examiner |
|---|---|---|
| The Hague | 4 July 2019 | Oancea, A |

EPO FORM 1503 03.82 (P04C01)

## ANNEX TO THE EUROPEAN SEARCH REPORT
## ON EUROPEAN PATENT APPLICATION NO.

EP 19 16 3299

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2019

| Patent document cited in search report | Publication date | Patent family member(s) | Publication date |
|---|---|---|---|
| US 2015088002 A1 | 26-03-2015 | US 2015087928 A1<br>US 2015088002 A1<br>US 2015088003 A1<br>US 2015088431 A1<br>WO 2015042484 A1 | 26-03-2015<br>26-03-2015<br>26-03-2015<br>26-03-2015<br>26-03-2015 |
| US 2013144136 A1 | 06-06-2013 | US 2013144136 A1<br>US 2015313541 A1 | 06-06-2013<br>05-11-2015 |
| US 2015289820 A1 | 15-10-2015 | NONE | |
| US 2017360316 A1 | 21-12-2017 | CN 109195524 A<br>US 2017360316 A1<br>WO 2017218097 A1 | 11-01-2019<br>21-12-2017<br>21-12-2017 |

EPO FORM P0459

For more details about this annex : see Official Journal of the European Patent Office, No. 12/82

**EP 3 539 473 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2004051 A1
- GB 201209340 A
- US 7033321 B1
- US 20130144136 A1
- WO 2002015787 A1